# EUROPÄISCHE PATENTSCHRIFT

(11) **EP 2 674 399 B1**
(45) Veröffentlichungstag und Bekanntmachung des Hinweises auf die Patenterteilung: **14.05.2014**
(21) Anmeldenummer: 12004438.3
(22) Anmeldetag: 13.06.2012
(51) Int. Cl.: C02F 1/44, C02F 103/02, C02F 103/04, C02F 1/68

(54) **Vorrichtung zur Erzeugung von Reinstwasser nach dem Prinzip der Umkehrosmose**
Device for producing ultra-pure water according to the principle of reverse osmosis
Dispositif de génération d'eau pure selon le principe d'osmose inverse

(43) Veröffentlichungstag der Anmeldung: 18.12.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A1- 1 862 213
- EP-A1- 2 423 169
- WO-A1-99/55448
- DE-A1- 19 530 190

## Beschreibung

Die Erfindung betrifft eine Vorrichtung zur Erzeugung von Reinstwasser nach dem Prinzip einer RO (Umkehrosmose), mit einem RO-Filtermodul, der durch eine Membran in einen Primärraum und einen Sekundärraum unterteilt ist, mit einem atmosphärisch belüfteten Vorlaufbehälter, in den eine Rohwasserzuflussleitung einmündet, wobei von dem unteren Ende des Vorlaufbehälters eine Leitung, in die eine Pumpe eingeschaltet ist, zu dem Primärraum führt, wobei von dem Primärraum eine Konzentratrücklaufleitung zu dem Vorlaufbehälter zurück führt und von dem Sekundärraum eine Permeatleitung ausgeht, an die Dialysegeräte anschließbar sind.

Umkehrosmoseanlagen dieser Art dienen zur Gewinnung reinen, keimfreien Wassers aus Leitungswasser z.B. für medizinische und lebensmitteltechnische Anwendungen. Ihr Funktionsprinzip besteht darin, dass das aufzubereitende Wasser in einem Filtermodul unter hohem Druck an der Oberfläche einer semipermeablen Membran entlang geführt wird, wobei ein Teil des Wassers, das Permeat, durch die Membran tritt, und auf der anderen Seite der Membran gesammelt und der Verbrauchsstelle, z.B. einem oder mehrerer Dialysegeräten, zugeführt wird. Der nicht durch die Membran tretende, mit zurückgehaltenen Stoffen angereicherte Teil des Rohwassers, das Konzentrat, fließt am Ende der Strömungsstrecke des Primärraums aus dem Filtermodul aus. Um den Wasserverbrauch der Umkehrosmoseanlage möglichst gering zu halten, wird ein Teil des Konzentrates meist wieder dem Vorlaufbehälter zugeführt.

Das Leitungssystem, insbesondere das mit dem zu dem Vorlaufbehälter zurückgeführten Konzentrat, ist nicht frei von organischen Verunreinigungen. Ohne besondere Gegenmaßnahmen kann es daher zu einer Besiedelung dieses Flüssigkeitssystems, d.h. des Vorlaufbehälters und der zu dem Filtermodul führenden und von dort zu dem Vorlaufbehälter zurück führenden Leitungen kommen. Dabei würde sich ein sogenannte Biofilm auf den Innenflächen des flüssigkeitsführenden Systems bilden.

Um dies zu verhindern, ist es notwendig, in geeigneten Zeitabständen eine Desinfektion dieses Teils des Flüssigkeitssystems der Umkehrosmoseanlage durchzuführen. Hierzu wird der normale Betrieb unterbrochen und dem flüssigkeitsführenden System ein chemisches Desinfektionsmittel zugeführt. Nach einer geeigneten Einwirkungszeit erfolgt ein Spülvorgang, der dazu dient, das eingebrachte Desinfektionsmittel und seine Reaktionsprodukte wieder zu entfernen, so dass anschließend der normale Versorgungsbetrieb wieder aufgenommen werden kann.

Wegen der erheblichen Gefahren, die mit einer unkontrollierten Zufuhr eines Desinfektionsmittels verbunden wären, insbesondere bei Anwendung im medizinischen Bereich (Hämodialyse), werden alle für die Durchführung der Desinfektion notwendigen Arbeitsschritte bisher unter vollständiger Kontrolle des Bedienungspersonals manuell durchgeführt. Hierzu zählt u.a. das Herstellen und spätere Trennen einer Verbindungsleitung mit einem externen Desinfektionsmittelbehälter und die Überwachung der korrekten Zufuhr des Desinfektions-mittels.

Aus der EP 1 862 213 A1 ist eine Vorrichtung zur Desinfektion einer Umkehrosmoseanlage bekannt, bei der eine Pumpe das über eine Saugleitung aus einem atmosphärisch belüfteten Vorratsbehälter entnommene chemische Desinfektionsmittel in das Leitungssystem fördert. Dabei ist eine Ansaugkammer in die Saugleitung eingefügt, die einen oberen Anschluss zu der Pumpe, einen unteren Anschluss zu dem Vorratsbehälter und eine mit dem oberen Teil verbundene Belüftungsleitung hat, die mit einem Absperrorgan versehen ist, das im Betriebszustand der Desinfektionsmittelzufuhr geschlossen und während der übrigen Betriebszustände der Umkehrosmoseanlage geöffnet ist.

Aufgabe der vorliegenden Erfindung ist es, die Durchführung einer Desinfektion der betrachteten Art weitgehend zu automatisieren.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass in die Konzentratrücklaufleitung eine Venturipumpe bzw. ein Venturirohr mit der Konvergenzkammer und der Divergenzkammer eingesetzt ist, wobei an der Saugöffnung der Venturipumpe ein Ansaugschlauch angebracht ist, der mittels einer Kupplung entweder stromaufwärts der Venturipumpe und vorzugsweise eines Strömungswiderstands mit der Konzentratrück-laufleitung oder mit einem externen Behälter verbindbar ist, der ein Desinfektionsmittel oder ein Reinigungsmittel enthält. Die Kupplung ist bevorzugt eine Steckkupplung, die zwei ineinander steckbare Kupplungsteile enthält, wobei der stationäre Teil der Kupplung sich bei ihrer Trennung durch Federkraft selbsttätig schließt.

Dabei wird mit großem Vorteil vorgeschlagen, dass an dem Behälter und an der Konzentratrücklaufleitung je ein stationäres Kupplungsteil und an dem Ende des Ansaugschlauchs ein zugehöriges Gegenkupplungsteil angeordnet sind. Die stationären Kupplungsteile haben bevorzugt jeweils einen Reedkontakt, und an dem Gegenkupplungsteil befindet sich ein Ringmagnet, so dass bei der Konnektion und bei der Diskonnektion des Ansaugschlauchs der so gebildete Reedschalter einen Schaltvorgang ausführt, der als Steuersignal für die Umkehrosmoseanlage verwendet wird.

Im Normalbetrieb der RO ist der Ansaugschlauch an die Konzentratrücklaufleitung angeschlossen, indem die beiden Kupplungsteile ineinander eingesteckt sind und der Reedschalter geschlossen ist. Ein Teil des Konzentrats durchfließt infolge des Strömungswiderstands stromabwärts der Abzweigung den Ansaugschlauch, wodurch dieser ständig gespült wird.

Wenn eine Desinfektion vorgenommen werden soll, wird die Steckkupplung an der Konzentratrücklaufleitung gelöst, was einen Schaltvorgang des Reedschalters zur Folge hat. Dieser Schaltvorgang wird mit großem Vorteil als Steuersignal verwendet, um automatisch den normalen Betrieb zu unterbrechen. Wenn das Kupplungselement am Ende des flexiblen Saugschlauchs anschließend mit dem Kupplungselement an dem externen Behälter zusammen gesteckt wird, kann - wenn hier ein entsprechender Reedschalter angeordnet ist - ein Schaltvorgang des Reedschalters erfasst werden, woraufhin vorzugsweise automatisch ein Desinfektionsvorgang eingeleitet wird. Dabei kann beispielsweise eine vorbestimmte Zeitspanne lang Desinfektionsmittel in die Venturipumpe eingesaugt werden, oder dieser Saugvorgang erfolgt so lange, bis eine in dem Primär- und/oder im Sekundär-Kreislauf angeordnete Analysevorrichtung eine vorbestimmte Konzentration des Desinfektionsmittels in der zu reinigenden Flüssigkeit erfasst. Es ist auch eine volumetrische Zudosierung, die von Niveauschaltern in Vorlaufbehältern überwacht wird, möglich. Nach einer geeigneten Einwirkungszeit folgt dann nach dem erneuten Umstecken des Ansaugschlauchs bevorzugt automatisch ein Spülvorgang, mit dem das eingebrachte Desinfektionsmittel und seine Reaktionsprodukte wieder entfernt werden, so dass anschließend der normale Versorgungsbetrieb wieder aufgenommen werden kann.

Mit der vorliegenden Erfindung ist eine weitgehend automatisierte Durchführung der Desinfektion ermöglicht, wobei die sicherheitstechnischen Vorraussetzung erheblich verbessert sind.

Der atmosphärisch belüftete Vorlaufbehälter ist besonders anfällig dafür, mit Mikroorganismen besiedelt zu werden. In diesen Vorlaufbehälter münden - in der Regel durch dessen Deckel - sowohl die Rohwasserzuflussleitung als auch die Konzentratrücklaufleitung und meist auch eine Permeatrücklaufleitung ein, die den Vorlaufbehälter teilweise füllen. Das Füllniveau wird in der Regel durch eine Einrichtung zur Niveauerkennung des jeweiligen Füllstandes erfasst.

Um eine Besiedelung der Innenwand des Vorlaufbehälters mit einem Biofilm zu vermeiden oder bereits abgelagerte Mikroorganismen zu beseitigen, wird nach einem anderen Gesichtspunkt der vorliegenden Erfindung, der unabhängig von der Art der Einbringung des Desinfektionsmittels in die Konzentratrücklaufleitung ist, vorgeschlagen, dass an oder unter der Einmündung der in den Vorlaufbehälter einmündenden Leitungen eine Verteilungseinrichtung oder Ablenkeinrichtung für die Flüssigkeitsstrahlen angeordnet ist, die die in den Vorlaufbehälter eintretende Flüssigkeit gegen die Innenwand des Vorlaufbehälters richtet, so dass die Flüssigkeit entlang der Innenwand des Vorlaufbehälters nach unten abläuft. Hierdurch werden organische Ablagerungen an der Innenwand des Vorlaufbehälters zuverlässig verhindert und die Wandflächen des Vorlaufbehälters vollständig ausgespült.

Bei der Verteilungseinrichtung bzw. Ablenkeinrichtung kann es sich um einen im wesentlichen konisch nach außen abfallenden Verteilungsschirm geeigneter Größe handeln oder beispielsweise um zerstäubende Düsen, die Flüssigkeitsstrahlen gegen die Innenwand des Vorlaufbehälters richten.

Es kann auch vorgesehen sein, dass unter der Einmündung der Wasserzuflussleitung, der Konzentratrücklaufleitung und der Permeatrücklaufleitung in den Vorlaufbehälter je eine der oben genannten Verteilungseinrichtungen oder - bevorzugt - ein gemeinsamer im wesentlichen konischer Verteilungsschirm angeordnet sind.

Nach einem weiteren unabhängigen Gesichtspunkt der vorliegenden Erfindung kann die Venturipumpe aus wenigstens zwei Blöcken mit Fließkanälen zusammen gesetzt sein, die dicht miteinander - bevorzugt unter Zwischenschaltung von O-Ringdichtungen - verbunden sind. Die Blöcke bestehen vorzugsweise aus einem harten Kunststoff. Eine derartige Modulbauweise der Venturipumpe ermöglicht die kostengünstige Herstellung von Venturipumpen unterschiedlicher Größen und Formen durch Zusammensetzen von vorgefertigten Elementen nach dem Baukastenprinzip.

Nach einem weiteren Vorschlag der Erfindung ist eine derartig zusammen gesetzte Venturipumpe an dem Deckel des Vorlaufbehälters befestigt.

Die Erfindung wird nachfolgend mit Bezug auf die beigefügten Zeichnungen näher beschrieben. Dabei zeigen:
- Figur 1: das Schema einer erfindungsgemäßen RO-Anlage;
- Figur 2: den Bereich der Venturipumpe der Figur 1 in vergrößerter Darstellung;
- Figur 3: den Teilbereich der Einmündung der Konzentratrücklaufleitung in den Vorlaufbehälter mit an dessen Deckel befestigter Venturipumpe mit großem Verteilerschirm und
- Figur 4: eine ähnliche Darstellung mit kleinem Verteilerschirm.

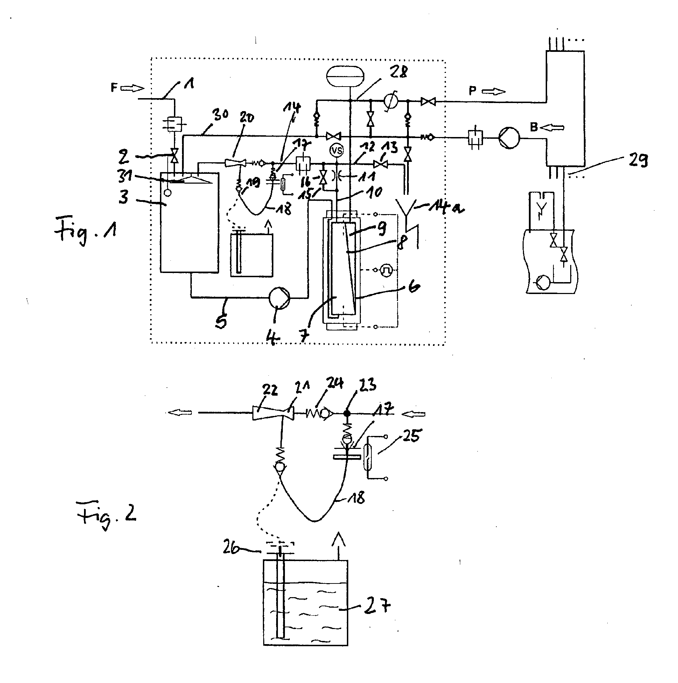

Es wird zunächst auf Figur 1 Bezug genommen. Durch eine Leitung 1 wird Rohwasser zugeführt, das über ein Einlassventil 2 in den Vorlaufbehälter 3 gelangt. Die Flüssigkeit in dem Vorlaufbehälter 3 wird mittels einer Pumpe 4 durch eine Leitung 5 einem RO-Filtermodul 6 zugeführt, dessen Primärraum 7 durch eine Membran 8 von dem Sekundärraum 9 getrennt ist.

Die Flüssigkeit tritt dabei zunächst in einen umlaufenden Ringraum am oberen Randbereich des Modulrohres ein und strömt dann entlang der gesamten Innenwand nach unten, so dass das Filtermodul Totraum-frei ist und sich darin kein Biofilm ablagern kann.

Aus dem Primärraum 7 wird Konzentrat durch eine Leitung 10 abgeführt, in der eine Drossel 11 für den im Primärraum 7 herrschenden Druck sorgt. Das Konzentrat kann entweder durch eine Leitung 12, in die ein Ventil 13 eingeschaltet ist, in einen Abfluss 14a abgeführt werden, oder durch eine Konzentratrücklaufleitung 14 in den Vorlaufbehälter 3 zurückgeführt werden. Die Drossel 11 ist durch eine Bypassleitung 15 mit einem Ventil 16 überbrückbar, um zum einen die Strömung durch die Leitung 14 beim Ansaugen von Desinfektionsmittel zu vergrößern oder auch um Rückstände der Primärseite zum Abfluss 14a abzuführen.

An der Konzentratrücklaufleitung 14 ist eine Kupplung 17 angeordnet, die aus einem stationären Kupplungselement, das fest mit der Leitung 14 verbunden ist, und einem Gegenkupplungselement zusammensetzbar ist, das am Ende eines flexiblen Ansaugschlauchs 18 angebracht ist. In den Ansaugschlauch 18 ist ein Rückschlagventil 19 eingeschaltet. Der Ansaugschlauch 18 ist an der Saugöffnung einer Venturipumpe 20 befestigt, deren Konvergenzkammer 21 und Divergenzkammer 22 in die Leitung 14 eingeschaltet sind. Zwischen der Venturipumpe 20 und der Abzweigstelle 23 ist ein Strömungswiderstand 24 angeordnet, mit der Folge, dass bei geschlossener Kupplung 17 im Normalbetrieb der RO-Anlage, eine Teilmenge des Konzentrats durch den Ansaugschlauch 18 zur Venturipumpe 20 fließt.

Das an der Leitung 14 stationär befestigte Kupplungsteil ist mit einem Reedkontakt versehen, während das am Ende des Ansaugschlauches 18 angebrachte Gegenkupplungsteil einen Ringmagneten trägt. Die beiden Bauteile bilden zusammen einen Reedschalter 25, dessen Schaltstellung eine Steuerungs- und Überwachungsfunktion für die RO-Anlage hat. Wenn die Kupplung 17 geöffnet, d.h. der Ansaugschlauch 18 entfernt wird, wird der normale Betrieb der RO-Anlage automatisch unterbrochen.

Das Gegenkupplungselement am Ende des Ansaugschlauchs 18 kann mit einem entsprechenden Kupplungselement 26 an einem externen Behälter 27 zusammengesteckt werden, der ein Desinfektionsmittel oder Reinigungsmittel enthält. Auch hier kann ein entsprechender Reedschalter ein das Schließen der Kupplung anzeigendes Steuersignal abgeben. Damit kann ein automatisches Desinfektionsprogramm ausgelöst werden.

Aus der Sekundärkammer 9 des RO-Filtermoduls 6 fließt Permeat durch eine Ringleitung 28 zu Entnahmestellen 29 für Dialysegeräte. Nicht benötigtes Permeat fließt durch eine Permeatrücklaufleitung 30 in den Vorlaufbehälter 3 zurück.

Unter den Einlauföffnungen der Wasserzuflussleitung 1, der Permeatrücklaufleitung 30 und der Konzentratrücklaufleitung 12 ist ein im wesentlichen konischer Verteilerschirm 31 angeordnet, auf den die zugeführten Flüssigkeiten fallen, die von dem Verteilerschirm 31 gegen die Innenwand des Vorlaufbehälters 3 gerichtet werden. Damit strömt ständig Flüssigkeit an der Innenwand des Vorlaufbehälters 3 herab, so dass sich kein Biofilm dort ablagern kann. Bei jedem Desinfektionsvorgang wird zudem mitgeführtes Desinfektionsmittel an die Innenwand geführt. Im nachfolgenden Ausspülprogramm wird das Desinfektionsmittel rückstandsfrei von der Innenoberfläche des Vorlaufbehälters enfernt.

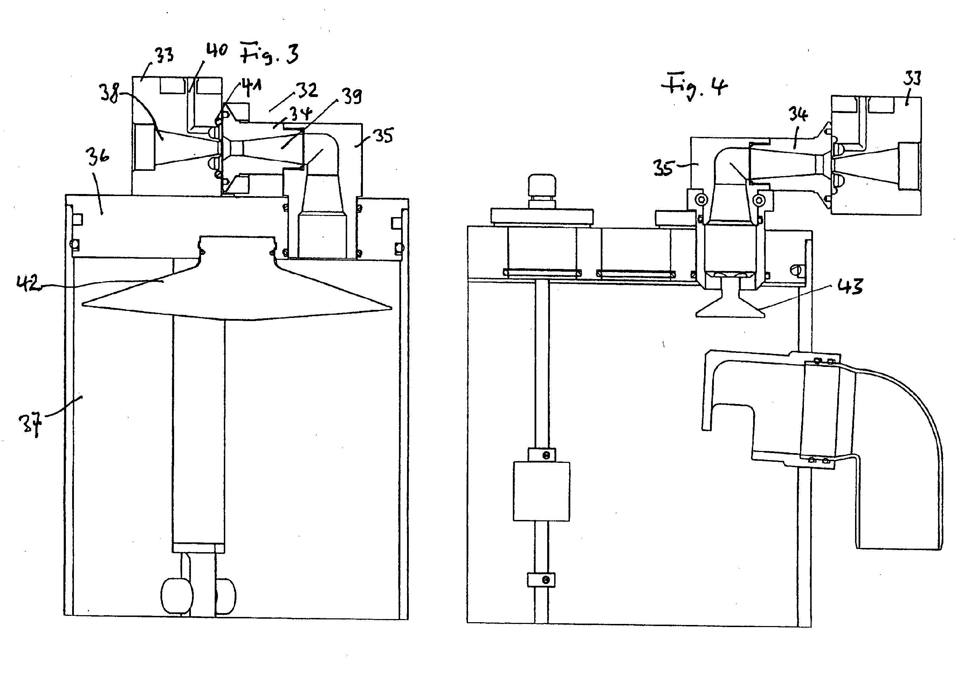

Figur 3 zeigt, dass eine Venturipumpe 32 aus zwei Blöcken 33 und 34 zusammengesetzt sein kann, wobei ein weiterer Block 35 den Konzentrateinlauf durch einen Deckel 36 des Vorlaufbehälters 37 bildet. Die Blöcke 33 bis 35 bestehen bevorzugt aus einem harten Kunststoff. Die Konvergenzkammer 38 ist mit der Divergenzkammer 39 zusammengesetzt, und in den mittigen Ansaugbereich mündet ein Saugkanal 40 ein. Die Blöcke 33 bis 35 sind mit geeigneten Mitteln unter Zwischenschaltung von O-Dichtungsringen 41 aneinander befestigt. Unter dem Deckel 36 ist ein im wesentlichen konischer Ablenk-/Verteilerschirm 42 befestigt, der die auftreffende Flüssigkeit gegen die Innenwand des Vorlaufbehälters 37 richtet.

Bei der in Figur 4 dargestellten Ausführungsform ist am Einlauf der Konzentratrücklaufleitung ein kleiner Ablenkschirm 43 beispielhaft für alle in den Vorlaufbehälter einmündenden Flüssigkeiten zu demselben Zweck angeordnet.

## Patentansprüche

1. Vorrichtung zur Erzeugung von Reinstwasser nach dem Prinzip einer RO (Umkehrosmose), mit einem RO-Filtermodul (6), der durch eine Membran (8) in einen Primärraum (7) und einen Sekundärraum (9) unterteilt ist,
mit einem atmosphärisch belüfteten Vorlaufbehälter (3), in den eine Wasserzuflussleitung (1) einmündet, wobei von dem unteren Ende des Vorlaufbehälters (3) eine Leitung (5), in die eine Pumpe (4) eingeschaltet ist, zu dem Primärraum (7) führt,
wobei von dem Primärraum (7) eine Konzentratrücklaufleitung (14) zu dem Vorlaufbehälter (3) zurück führt und
von dem Sekundärraum (9) eine Permeatleitung (28) ausgeht, an die Dialysegeräte anschließbar sind,
**dadurch gekennzeichnet,**
**dass** in die Konzentratrücklaufleitung (14) eine Venturipumpe (20) mit ihrer Konvergenzkammer (21) und Divergenzkammer (22) eingesetzt ist, in die ein Ansaugschlauch (18) einmündet, der wahlweise mittels einer Steckkupplung (17,26) mit bei ihrer Trennung selbsttätig schließenden Kupplungsteilen an den stationären Teilen mit einem Desinfektionsmittel und/oder Reinigungsmittel enthaltenden Behälter (27) oder stromaufwärts der Venturipumpe (20) mit der Konzentratrücklaufleitung (14) verbindbar ist.

2. Vorrichtung nach Anspruch 1,
**dadurch gekennzeichnet,**
**dass** eine Permeatrücklaufleitung (30) in den Vorlaufbehälter (3) einmündet.

3. Vorrichtung nach einem der Ansprüche 1 oder 2,
**dadurch gekennzeichnet,**
**dass** stromaufwärts der Venturipumpe (20) ein Strömungswiderstand (24) in die Konzentratrücklaufleitung (14) eingeschaltet ist.

4. Vorrichtung nach einem der Ansprüche 1 bis 3,
**dadurch gekennzeichnet,**
**dass** an dem Behälter (27) und an der Konzentratrücklaufleitung (14) je ein stationäres Kupplungsteil und an dem Ende des Ansaugschlauchs (18) ein zugehöriges Gegenkupplungsteil angeordnet sind.

5. Vorrichtung nach Anspruch 4,
**dadurch gekennzeichnet,**
**dass** an den stationären Kupplungsteilen je ein Reedkontakt und an dem Gegenkupplungsteil ein Ringmagnet angeordnet ist und dass die Konnektion bzw. Diskonnektion des Ansaugschlauches (18) erfasst und als Steuersignal der RO verwendet wird.

6. Vorrichtung nach einem der Ansprüche 1 bis 5,
**dadurch gekennzeichnet,**
**dass** in den Ansaugschlauch (18) ein Rückschlagventil (19) eingeschaltet ist.

7. Vorrichtung nach einem der Ansprüche 1 bis 6,
**dadurch gekennzeichnet,**
**dass** an oder unter der Einmündung der Konzentratrücklaufleitung (14) in den Vorlaufbehälter (3, 37) eine Verteilungseinrichtung (31, 42, 43) oder Ablenkeinrichtung für den Flüssigkeitsstrahl angeordnet ist, die die eintretende Flüssigkeit gegen die Innenwand des Vorlaufbehälters (3, 37) richtet.

8. Vorrichtung nach Anspruch 7,
**dadurch gekennzeichnet,**
**dass** die Verteilungseinrichtung eine Düseneinrichtung und/oder ein im wesentlichen konischer Verteilungsschirm ist.

9. Vorrichtung nach einem der Ansprüche 2 bis 8,
**dadurch gekennzeichnet,**
**dass** unter der Einmündung der Wasserzuflussleitung (1), der Konzentratrücklaufleitung (14) und der Permeatrücklaufleitung (30) in den Vorlaufbehälter (3,37) je eine Verteilungseinrichtung oder ein gemeinsamer im wesentlichen konischer Verteilungsschirm angeordnet ist.

10. Vorrichtung nach einem der Ansprüche 1 bis 9,
**dadurch gekennzeichnet,**
**dass** die Venturipumpe aus wenigstens zwei Blöcken (33,34) mit Fließkanälen (38,39,40) zusammengesetzt ist, die dicht miteinander verbunden sind.

11. Vorrichtung nach Anspruch 10,
**dadurch gekennzeichnet,**
**dass** die Venturipumpe an einem Deckel (36) des Vorlaufbehälters (37) befestigt ist.

## Claims

1. Apparatus for producing highly pure water in accordance with the RO (reverse osmosis) principle including an RO filter module (6), which is divided by a membrane (8) into a primary space (7) and a secondary space (9), an atmospherically vented feed tank (3), into which a water supply conduit (1) discharges, whereby a conduit (5), into which a pump (4) is connected, leads from the lower end of the feed tank (3) to the primary space (7), whereby a concentrate return conduit (14) leads back from the primary space (7) to the feed tank (3) and a permeate line (28), to which dialysis devices may be connected, extends out from the secondary space (9), **characterised in that** inserted into the concentrate return conduit (14) there is a venturi pump (20) with its convergence chamber (21) and divergence chamber (22), communicating with which there is a suction hose (18), which is selectively connectable by means of a plug coupling (17, 26) with coupling components, which close automatically when they are disconnected, on the stationary parts with a container (27) containing a disinfectant and/or cleaning agent or with the concentrate return conduit (14) upstream of the venturi pump (20).

2. Apparatus as claimed in Claim 1, **characterised in that** a permeate return conduit (30) communicates with the feed tank (3).

3. Apparatus as claimed in one of Claims 1 or 2, **characterised in that** a flow resistance (24) is connected into the concentrate return conduit (14) upstream of the venturi pump (20).

4. Apparatus as claimed in one of Claims 1 to 3, **characterised in that** arranged on the container (27) and on the concentrate return conduit (14) is a respective stationary coupling member and on the end of the suction hose (18) there is an associated complementary coupling member.

5. Apparatus as claimed in Claim 4, **characterised in that** arranged on the stationary coupling members there is a respective Reed contact and on the complementary coupling member there is a ring magnet and that the connection and disconnection of the suction hose (18) is detected and used as a control signal for the RO.

6. Apparatus as claimed in one of Claims 1 to 5, **characterised in that** connected into the suction hose (18) there is a non-return valve (19).

7. Apparatus as claimed in one of Claims 1 to 6, **characterised in that** arranged on or beneath the communication of the concentrate return conduit (14) with the supply tank (3, 37) there is a distribution device (31, 42, 43) or deflecting device for the liquid jet, which directs the entering liquid against the inner wall of the feed tank (3, 37).

8. Apparatus as claimed in Claim 7, **characterised in that** the distributor device is a nozzle device and/or a substantially conical distribution screen.

9. Apparatus as claimed in one of Claims 2 to 8, **characterised in that** arranged beneath the communication of the water supply conduit (1), the concentrate return conduit (14) and the permeate return conduit (30) with the feed tank (3, 37) there is a respective distribution device or a common, substantially conical distribution screen.

10. Apparatus as claimed in one of Claims 1 to 9, **characterised in that** the venturi pump is composed of at least two blocks (33, 34) with flow channels (38, 39, 40), which are connected together in a sealed manner.

11. Apparatus as claimed in Claim 10, **characterised in that** the venturi pump is fastened to a lid (36) of the feed tank (37).

## Revendications

1. Dispositif de génération d'eau pure selon le principe d'une osmose inverse, comprenant un module de filtrage d'osmose inverse (6), qui est divisé par une membrane (8) en un espace primaire (7) et un espace secondaire (9),
comprenant un premier bassin de décantation (3) sous pression atmosphérique, dans lequel débouche une conduite d'amenée d'eau (1), sachant qu'une conduite (5) dans laquelle est installée une pompe (4) mène depuis l'extrémité inférieure du premier bassin de décantation (3) vers l'espace primaire (7),
sachant qu'une conduite de retour de concentré (14) part de l'espace primaire (7) pour revenir dans le premier bassin de décantation (3) et
qu'une conduite de perméat (28) part de l'espace secondaire (9), les appareils de dialyse peuvent être raccordés à ladite conduite de perméat,
**caractérisé en ce que** :
une pompe Venturi (20) est introduite par sa chambre de convergence (21) et sa chambre de divergence (22) dans la conduite de retour de concentré (14), un tuyau flexible d'aspiration (18) débouchant dans ladite pompe Venturi, lequel tuyau flexible d'aspiration pouvant être relié au choix, au moyen d' un système d'accouplement par emboîtement (17, 26), à l'aide de parties d'accouplement se fermant automatiquement lors de leur séparation, au niveau de parties stationnaires, à un réservoir (27) contenant des agents de désinfection et/ou des agents de nettoyage ou, en amont, à la pompe Venturi (20) par la conduite de retour de concentré (14).

2. Dispositif selon la revendication 1,
**caractérisé en ce que** :
une conduite de retour de perméat (30) débouche dans le premier bassin de décantation (3).

3. Dispositif selon l'une quelconque des revendications 1 ou 2,
**caractérisé en ce que** :
en amont de la pompe Venturi (20) est installée une résistance d'écoulement (24) dans la conduite de retour de concentré (14).

4. Dispositif selon l'une quelconque des revendications 1 à 3,
**caractérisé en ce que** :
respectivement une partie d'accouplement stationnaire est disposée au niveau du réservoir (27) et au niveau de la conduite de retour de concentré (14), et **en ce qu'**une partie d'accouplement complémentaire associée est disposée au niveau de l'extrémité du tuyau flexible d'aspiration (18).

5. Dispositif selon la revendication 4,
**caractérisé en ce que** :
respectivement un contact Reed est disposé au niveau des parties d'accouplement stationnaires et un aimant annulaire est disposé au niveau de la partie d'accouplement complémentaire, et **en ce que** la connexion ou la déconnexion du tuyau flexible d'aspiration (18) est détectée et est utilisée comme signal de commande de l'osmose inverse.

6. Dispositif selon l'une quelconque des revendications 1 à 5,
**caractérisé en ce que** :
une soupape de non-retour (19) est installée dans le tuyau flexible d'aspiration (18).

7. Dispositif selon l'une quelconque des revendications 1 à 6,
**caractérisé en ce que** :
un système de répartition (31, 42, 43) ou un système de déviation pour le jet de fluide est disposé au niveau de l'embouchure ou sous l'embouchure de la conduite de retour de concentré (14) menant dans le premier bassin de décantation (3, 37), ledit système de répartition ou de déviation orientant le liquide entrant contre la paroi intérieure du premier bassin de décantation (3, 37).

8. Dispositif selon la revendication 7,
**caractérisé en ce que** :
le système de répartition est un système de buses et/ou un écran de répartition essentiellement conique.

9. Dispositif selon l'une quelconque des revendications 2 à 8,
**caractérisé en ce que** :
respectivement un système de répartition ou un écran de répartition commun essentiellement conique est disposé sous l'embouchure de la conduite d'amenée d'eau (1), de la conduite de retour de concentré (14) et de la conduite de retour de perméat (30) menant dans le premier bassin de décantation (3, 37).

10. Dispositif selon l'une quelconque des revendications 1 à 9,
**caractérisé en ce que** :
la pompe Venturi est composée d'au moins deux blocs (33, 34) dotés de canaux d'écoulement (38, 39, 40), qui sont reliés les uns aux autres de manière étanche.

11. Dispositif selon la revendication 10,
**caractérisé en ce que** :
la pompe Venturi est fixée à un couvercle (36) du premier bassin de décantation (37).
